# EUROPEAN PATENT APPLICATION

(11) **EP 4 843 583 A1**
(43) Date of publication of application: **25.11.2026**
(21) Application number: 25742263.4
(22) Date of filing: 17.01.2025
(51) Int. Cl.: A61K 38/48, A61K 47/26, A61K 47/18, A61P 17/00

(54) **RAPID EFFICACY EXPRESSION EFFECT OF NON-COMPLEXED BOTULINUM TOXIN COMPOSITION AND METHOD RELATED THERETO**

(30) Priority: 18.01.2024 KR 20240007727
(71) Applicant: Medytox Inc., Chungcheongbuk-do 28126 (KR)
(72) Inventor: KWAK, Seongsung, Suwon-si, Gyeonggi-do 16228 (KR); BYUN, Jae Yoon, Yongin-si, Gyeonggi-do 17095 (KR)
(74) Representative: Santarelli
(86) International application number: PCT/KR2025/000994
(87) International publication number: WO 2025/155124

(57) **Abstract**

A botulinum toxin composition for use in a method for treating a disease in a subject in need thereof, the composition being free of animal protein and comprising a non-complexed botulinum toxin as an active ingredient, wherein the method comprises locally administering a therapeutically effective amount of the composition to the subject in need thereof, and wherein the composition has a faster onset of efficacy than a composition containing animal protein and comprising a 19S botulinum toxin complex as an active ingredient.

## Description

### Technical Field

The present disclosure relates to a botulinum toxin formulation and methods related thereto, and more particularly, to a botulinum toxin formulation exhibiting a rapid onset effect and a cosmetic or therapeutic method using the same.

### Background Art

Botulinum neurotoxin (BoNT) is a polypeptide product of the anaerobic bacterium *Clostridium botulinum*, and functions to paralyze muscles by preventing the release of neurotransmitters at the neuromuscular junction. Although botulinum toxin is originally a toxic substance capable of causing lethality, it has been used for a variety of purposes with an expanded scope of application after its initial use in treating diseases caused by excessive muscle contraction, including strabismus. Botulinum toxin is primarily used to treat dystonia or other non-dystonic movement disorders such as hemifacial spasm, tremor, and spasticity. In addition, it is used for various indications such as ocular diseases, pain-related diseases including migraine, urologic diseases, and wrinkle treatment for cosmetic purposes.

Meanwhile, in the case of botulinum toxin serotype A, a several-day or longer period is required from the administration of an effective amount until the efficacy is manifested, thereby posing a problem in that an immediate onset of efficacy cannot be expected. Korean Patent Publication No. KR 10-2019-0126894 discloses the fast-acting nature of botulinum toxin serotype E; however, the botulinum toxin serotype E has a short duration of action, which increases the frequency of administration, thereby giving rise to a problem of deteriorating patient compliance.

### Disclosure of Invention

### Technical Problem

The present invention provides a botulinum toxin composition for use in a method for treating a disease in a subject in need thereof, or a method for treating a disease by administering the composition to a subject in need thereof, wherein the composition is free of animal protein and comprises a non-complexed botulinum toxin as an active ingredient, and wherein the composition has a faster onset of efficacy than a composition containing animal protein and comprising a 19S botulinum toxin complex as an active ingredient.

### Solution to Problem

According to an embodiment of the present invention, there is provided a botulinum toxin composition for use in a method for treating a disease in a subject in need thereof, the composition being free of animal protein and comprising a non-complexed botulinum toxin as an active ingredient, wherein the method comprises locally administering a therapeutically effective amount of the composition to the subject in need thereof, and wherein the composition has a faster onset of efficacy than a composition containing animal protein and comprising a 19S botulinum toxin complex as an active ingredient.

In one embodiment, a time to reach maximum DAS after administration of an effective amount of the composition is shorter than a time to reach maximum DAS after administration of an effective amount of the 19S botulinum toxin complex composition containing animal protein.

In another embodiment, a time to reach time to reach maximum DAS after administering the 19S botulinum toxin complex composition containing animal protein to a mouse at a dose of 12 U/kg is at least 40% longer than a time to reach maximum DAS after administering the composition to a mouse at a dose of 12 U/kg.

In another embodiment, the time to reach maximum DAS after administering the compositon to a mouse at a dose of 12U/kg is 20 hours to 30 hours.

In another embodiment, a time required to reach a level of 75% relative to a pre-administration CMAP value after administering the effective amount of the composition is shorter than a time required to reach a level of 75% relative to a pre-administration CMAP value after administering the effective amount of the 19S botulinum toxin complex composition containing animal protein.

In another embodiment, the time required to reach the level of 75% relative to the pre-administration CMAP value after administering the composition to a rat at a dose of 4 U/kg is less than 8 hours.

In another embodiment, an area under the curve of a CMAP graph for 72 hours after administration of the effective amount of the composition is smaller than an area under the curve of a CMAP graph for 72 hours after administration of an effective amount of the 19S botulinum toxin complex composition containing animal protein.

In another embodiment, the area under the curve of the CMAP graph for 72 hours after administering the composition to a rat at a dose of 4 U/kg is 1300 mV*hours or less.

In another embodiment, the composition may comprising the botulinum toxin, and further comprising one or more components selected from the group consisting of a surfactant, an amino acid, and an ionic compound. In another embodiment, the surfactant is at least one selected from the group consisting of polysorbate or poloxamer, and the amino acid may comprise methionine

In another embodiment, the composition for improving skin wrinkles, square jaw, pointed jaw, wounds, skin softening, scars, acne, pores, elasticity, or keloid symptoms, or for treating at least one disease selected from the group consisting of facial spasm, eyelid spasm, torticollis, blepharospasm, cervical dystonia, mid-pharyngeal dystonia, spasmodic dysphonia, migraine, pruritus ani, or hyperhidrosis.

In another embodiment, the botulinum toxin may comprise a botulinum toxin serotype A.

In another embodiment of the present invention, there is provided a method for preventing or treating a disease by administering the above-described composition to a subject in need thereof.

### Advantageous Effects of Invention

According to an embodiment of the present invention, there is provided a composition for preventing and treating a disease, comprising a non-complex botulinum toxin and having a rapid onset of action.

According to another embodiment of the present invention, there is provided a method for rapidly preventing or treating a disease by administering the composition to a subject in need thereof.

### Brief Description of Drawings

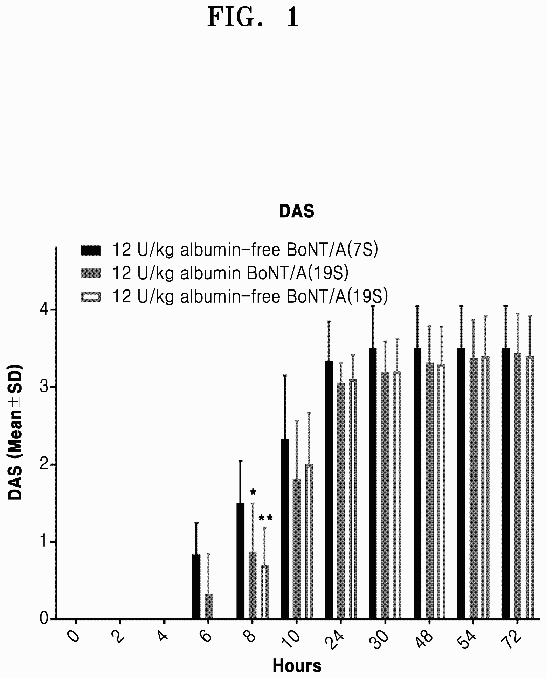

FIG. 1 is a graph showing changes over time in DAS values after administering three different formulations of botulinum toxin type A into the right gastrocnemius muscle of mice. The DAS values at each measurement time were analyzed using a t-test. *p<0.05, **p<0.01, vs. albumin-free BoNT/A(7S).

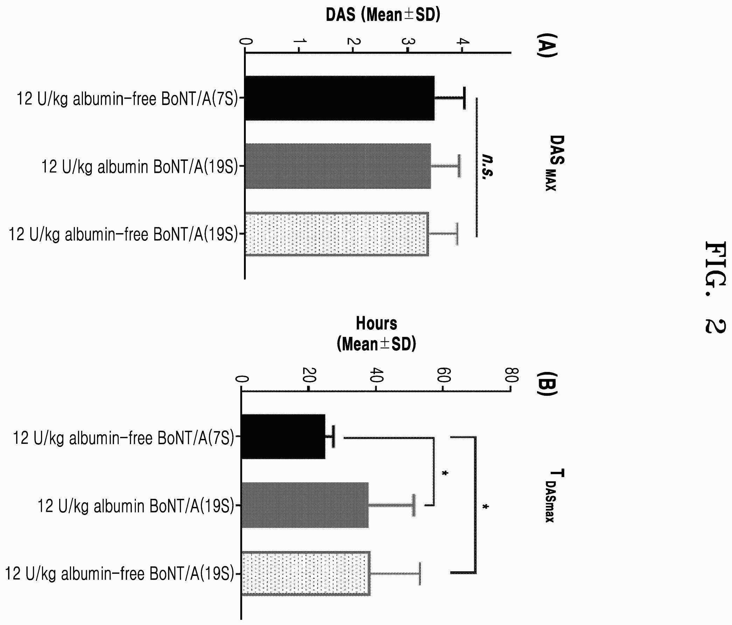

FIG. 2 is a graph showing a peak DAS score (A) and a time to reach the peak DAS score (B) after administering three different formulations of botulinum toxin type A into the right gastrocnemius muscle of mice. The analysis was performed using a t-test. *p<0.05 vs. albumin-free BoNT/A(7S). n.s. = not significant.

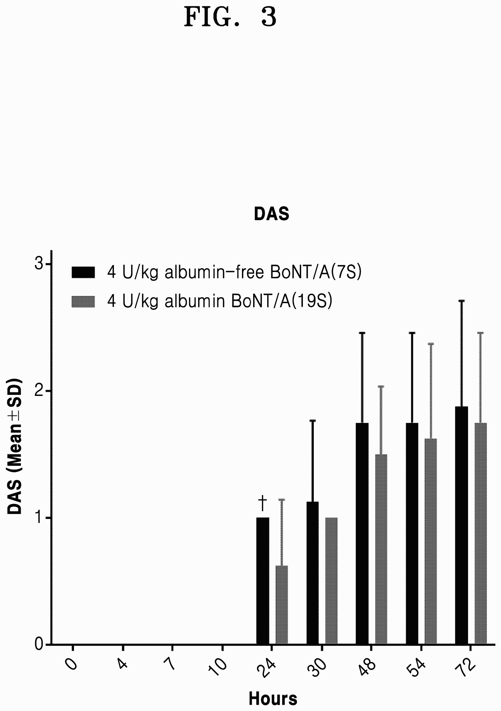

FIG. 3 is a graph showing changes over time in DAS values after administering two different formulations of botulinum toxin type A into the right gastrocnemius muscle of rats. The DAS values at each measurement time were analyzed using a t-test. ^{†}p=0.06 vs. albumin-free BoNT/A(7S).

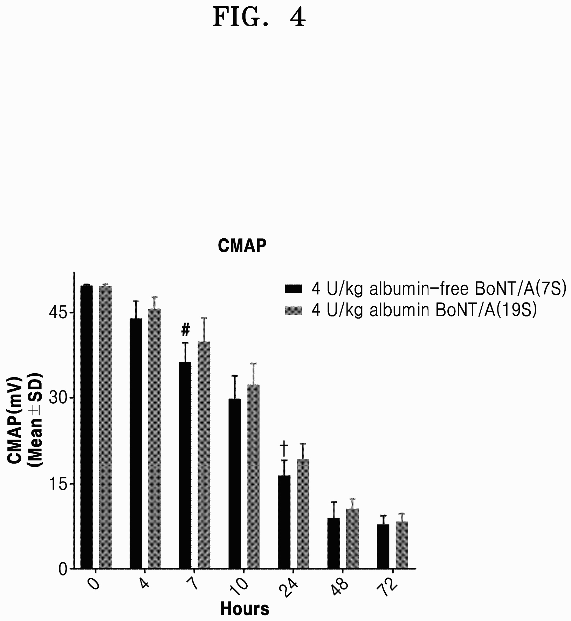

FIG. 4 is a graph showing changes over time in CMAP values after administering two different formulations of botulinum toxin type A into the right gastrocnemius muscle of rats. The CMAP values at each measurement time were analyzed using a t-test. ^{#}p = 0.08, ^{†}p = 0.05 vs. albumin-free BoNT/A(7S).

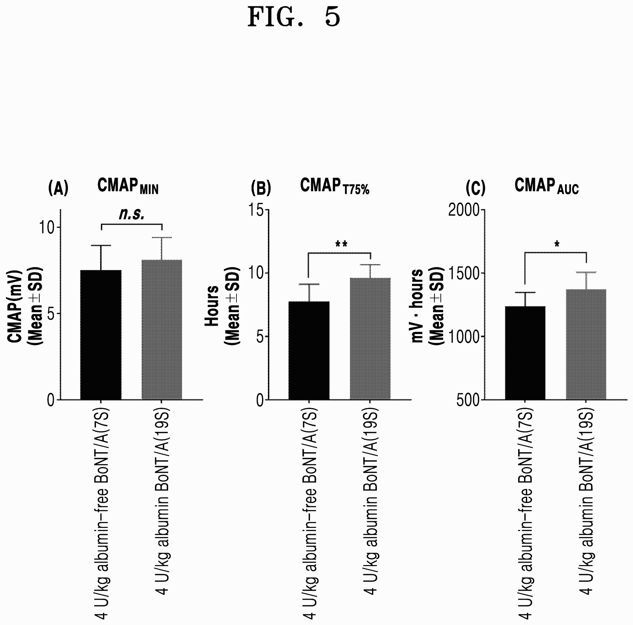

FIG. 5 is a graph showing a minimum CMAP value (A), a time to reach 75% of a baseline (pre-administration) CMAP (B), and a CMAP AUC (C) after administering two different formulations of botulinum toxin type A into the right gastrocnemius muscle of rats. The analysis was performed using a t-test. *p<0.05, **p<0.01 vs. albumin-free BoNT/A(7S). n.s. = not significant.

### Mode for the Invention

Unless otherwise defined, all technical and scientific terms used herein have the same meaning as commonly understood by one of ordinary skill in the art to which this technology belongs. For the purpose of understanding the present disclosure, the following definitions will apply, and singular forms include plural referents unless the context clearly dictates otherwise, and vice versa.

The term "and/or" includes any and all combinations that the associated components may define.

In interpreting components, they are interpreted to include an error range even if there is no separate explicit description. As used herein, the term "about" is used to include a conventional tolerance range in the relevant technical field. For example, the term "about" may mean that a tolerance of up to 10% exists in the corresponding numerical value or numerical range.

As used herein, the term 'botulinum toxin' refers to a botulinum toxin protein molecule produced by a bacterium or recombinantly, including all serotypes and variants or fusion proteins thereof.

In one aspect, the botulinum toxin may be used without limitation as to its serotype, and may be selected from the group consisting of serotype A (BoNT/A), serotype B (BoNT/B), serotype C (BoNT/C), serotype D (BoNT/D), serotype E (BoNT/E), serotype F (BoNT/F), serotype G (BoNT/G), serotype H (BoNT/H), serotype X (BoNT/X), *Enterococcus* species botulinum toxin J (eBoNT/J), and mosaic botulinum toxins and/or variants thereof. Examples of the mosaic toxins include BoNT/DC, BoNT/CD, and BoNT/FA. For example, type A may be used.

In one aspect, the botulinum toxin is derived from various BoNT/A subtypes, such as A1, A2, A3, A4, A5, A6, A7, A9, and A10; BoNT/B subtypes, such as B1, B2, B3, B4, B5, B6, B7, B8, Bnp, and Bbv; BoNT/C subtypes, such as C and CD; BoNT/D subtypes, such as D and DC; BoNT/E subtypes, such as E1, E2, E3, E4, E5, E6, E7, E8, and E9; BoNT/F subtypes, such as F1, F2, F3, F4, F5, F6, and F7; and BoNT/G subtypes, such as subtype G. The BoNT subtypes include chimeric BoNTs, such as BoNT/DC, BoNT/CD, BoNT/FA, and the like.

The botulinum toxin includes botulinum toxin derivatives. The botulinum toxin derivative may be a derivative comprising one or more chemical or functional modifications on a part or a part of a chain of a native botulinum toxin or a recombinant native botulinum toxin having botulinum toxin activity. For example, the botulinum toxin derivative may be a modified toxin having one or more amino acids deleted, modified, or substituted. The botulinum toxin may be a recombinant peptide, a fusion protein, or a hybrid neurotoxin prepared from, for example, subunits or domains of different toxin serotypes. The botulinum toxin may also be a part of a whole molecule having toxin activity, and may be utilized as a part of a combination or a conjugated molecule thereof, for example, a fusion protein.

The term "complex proteins" refers to hemagglutinin and a nontoxic nonhemagglutinin (NTNH) component lacking hemagglutinin activity. *Clostridium botulinum* strains produce immunologically distinct neurotoxins (types A to G). The molecular masses of types A to G neurotoxins (NTX) are about 150 kDa (7S). In a culture medium and food under acidic conditions, NTX binds to nontoxic components to form a large complex called progenitor toxins (PTX). Wherein "S" refers to Svedberg's sedimentation coefficient (Svedberg unit: 1S= 10^-13 seconds). PTX may have a molecular weight of 900 kDa (19S), 500 kDa (16S), and 300 kDa (12S). The 12S toxin consists of a neurotoxin (NTX) and a non-toxic non-hemagglutinin (NTNH) component lacking hemagglutinin activity, and the 19S and 16S toxins consist of NTX, NTNH, and hemagglutinin. Type A strains produce three forms of toxins (19S, 16S, and 12S), and types B, C, and D strains produce 16S and 12S toxins. Under alkaline conditions, PTX dissociates into NTX and nontoxic components, the 19S and 16S toxins dissociate into NTX and nontoxic components of NTNH and hemagglutinin, and the 12S toxin dissociates into NTX and NTNH.

The botulinum toxin composition of the present disclosure may be a complex botulinum toxin composition containing a complex protein. For example, the complex botulinum toxin may contain NTNH or hemagglutinin, in which case the molecular weight of the complex botulinum toxin may be about 900 kDa or 500 kDa.

The botulinum toxin composition of the present disclosure may be a non-complex botulinum toxin composition that does not contain a complex protein. For example, the non-complex botulinum toxin may not contain NTNH or hemagglutinin, in which case the molecular weight of the non-complex botulinum toxin may be about 150 kDa. In the present specification, the non-complex botulinum toxin may be referred to as a 7S botulinum toxin or a botulinum toxin having a molecular weight of 150 kDa.

The term "animal protein" refers to a product derived from blood, containing blood, or derived from other animals.

The botulinum toxin composition of the present disclosure may be a botulinum toxin composition that does not contain an animal protein. For example, the botulinum toxin composition of the present disclosure may not contain a protein stabilizer derived from an animal. In one embodiment, the botulinum toxin composition of the present disclosure may not comprise albumin, for example, human serum albumin or recombinant human albumin.

As used herein, the terms "unit," "unit(s)," or "U" refer to an LD₅₀ dose, which is defined as the amount of botulinum toxin that kills 50% of mice injected with the botulinum toxin, and are used interchangeably within a single product.

A "pharmaceutical composition" refers to a formulation containing an active ingredient. The term "formulation" means that at least one additional component, such as albumin (for example, human serum albumin or recombinant human albumin) and/or sodium chloride, is present in the pharmaceutical composition in addition to the active ingredient (e.g., botulinum toxin). The pharmaceutical composition is a formulation suitable for administration to a subject, such as a human patient. The pharmaceutical composition may be in a lyophilized form, for example, a solution formed after reconstitution of the lyophilized pharmaceutical composition using saline or water, or a solution form that does not require reconstitution. The pharmaceutical composition may be a liquid or a solid. The pharmaceutical composition may be free of animal-derived proteins and/or albumin.

The term "active ingredient" refers to a biologically or physiologically effective substance of a agent or composition. In particular, in the context of a pharmaceutical formulation or composition, the term "active ingredient" refers to a component substance that exhibits a target pharmaceutical effect. For example, the active ingredient of the present invention may be a non-complex botulinum toxin free of complex proteins.

"Administration" or "administering" refers to a step of providing a pharmaceutical composition or an active ingredient to a subject. The pharmaceutical composition may be administered through various suitable routes.

In one aspect, administration of the pharmaceutical composition may be performed by transdermal, subcutaneous, or intramuscular administration. In an embodiment, it may be performed by locally administering the composition to a muscle or a group of muscles. For example, reduction of forehead wrinkles or skin wrinkles may be achieved by administering the composition transdermally or subcutaneously to the corresponding wrinkles. For example, the composition of one embodiment may be administered by subcutaneous or intramuscular injection. The composition according to one embodiment may be administered by subcutaneous or intramuscular injection via a prefilled syringe. The composition of one embodiment may be transdermally administered using a transdermal patch or a microneedle.

A "pharmaceutical composition" refers to a formulation containing an active ingredient. The "pharmaceutical composition" may comprise at least one additional component, such as albumin (human serum albumin or recombinant human albumin) and/or sodium chloride, in addition to the active ingredient (e.g., botulinum toxin). The pharmaceutical composition is a formulation suitable for administration to a subject, such as a mammal including a human. The subject to which the composition of the present disclosure is administered may include, without limitation, a human or an animal, for example, a human, a pig, a dog, a cat, a cow, a horse, a mouse, and the like.

The pharmaceutical composition may be in a liquid form or a lyophilized form. When the pharmaceutical composition is a lyophilized formulation, the lyophilized pharmaceutical composition may be reconstituted using, for example, saline or water before use. It may be in a solution form. The pharmaceutical composition may be a liquid or a solid. The pharmaceutical composition may be free of animal-derived proteins and/or albumin.

"Treat," "treating," or "treatment" refers to alleviation or reduction (including partial reduction, substantial reduction, near-complete reduction, and complete reduction), resolution, or prevention (whether temporary or permanent) of a disease, disorder, or condition to achieve a desired therapeutic result, for example, by healing a wounded or damaged tissue, or by altering, changing, enhancing, ameliorating, improving, and/or beautifying an existing or perceived disease, disorder, or condition. In the present specification, "treatment" is a concept including prevention. "Prevention" refers to a delay in the onset of a disease, disorder, or condition. Prevention may be considered complete if the onset of the disease, disorder, or condition is delayed for a predetermined period of time.

In one embodiment, "treatment" refers to the treatment of a disease, disorder, or medical condition in a patient, such as a mammal (particularly, a human), comprising one or more of the following:
(a) preventing the occurrence of the disease, disorder, or medical condition, that is, preventing the recurrence of the disease or medical condition, or prophylactic treatment of a patient pre-disposed to the disease or medical condition;
(b) ameliorating the disease, disorder, or medical condition, that is, eliminating or causing regression of the disease, disorder, or medical condition in a patient, including antagonizing the effects of other therapeutic agents;
(c) suppressing the disease, disorder, or medical condition, that is, slowing or arresting the development of the disease, disorder, or medical condition in a patient; or
(d) relieving the symptoms of the disease, disorder, or medical condition in a patient.

As used herein, the terms "unit," "unit(s)," or "U" refer to an LD₅₀ dose, which is defined as the amount of botulinum toxin that kills 50% of mice injected with the botulinum toxin, and are used interchangeably within a single product.

In one aspect, a therapeutically effective amount of the botulinum toxin is about 0.01 U/kg to about 100 U/kg, about 0.1 U/kg to about 100 U/kg, about 0.2 U/kg to about 100 U/kg, about 0.2 U/kg to about 50 U/kg, about 0.2 U/kg to about 30 U/kg, about 0.2 U/kg to about 10 U/kg, about 0.2 U/kg to about 1 U/kg, about 1.2 U/kg, about 4 U/kg, or about 12 U/kg. In another aspect, based on an adult weighing 60 kg, the amount may be about 1 U to about 10,000 U, about 1 U to about 5,000 U, about 1 U to about 2,500 U, about 1 U to about 1,000 U, about 1 U to about 500 U, about 1 U to about 300 U, about 1 U to about 200 U, about 10 U to about 200 U, about 10 U to about 100 U, about 10 U to about 50 U, or about 10 U to 30 U.

According to an embodiment of the present invention, there is provided a botulinum toxin composition for use in a method for treating a disease of a subject in need thereof, comprising a non-complex botulinum toxin as an active ingredient without containing an animal protein, wherein the method comprises locally administering a therapeutically effective amount of the composition to the subject in need thereof, and wherein an onset of efficacy of the composition is faster onset of efficacy than a composition containing an animal protein and comprising a complex botulinum toxin as an active ingredient, wherein the complex botulinum toxin particularly includes a 19S complex botulinum toxin.

In the present invention, the expression "does not contain an animal protein" means that it is substantially free of protein derived from an animal.

The composition of one embodiment may be administered in a therapeutically effective amount, and as used herein, the term "effective amount" or "therapeutically effective amount" refers to an amount sufficient to treat or prevent a disease at a reasonable benefit/risk ratio applicable to any medical treatment or prevention. An effective dose level may be determined depending on factors known in the medical field, including a severity of the disease, an activity of the drug, an age, a body weight, a health status, and a gender of the patient, a sensitivity to the drug, a time of administration, a route of administration and an excretion rate of the composition of the present disclosure, a duration of treatment, and drugs used simultaneously or in combination with the composition of the present disclosure.

In one embodiment, the botulinum toxin may be administered in a single or multiple treatment sessions. The dose may be administered in a single or divided injection at an injection site. In the multiple treatment sessions, the botulinum toxin may be administered at intervals of 6 months, 4 months, or 3 months or less. In the multiple treatment sessions, the administration interval of the botulinum toxin includes a first treatment and a second treatment, and a dose of the second treatment may be less than, greater than, or equal to a dose of the first treatment.

A time to reach a maximum DAS (digit abduction score) after administering an effective amount of the composition may be shorter than a time to reach a maximum DAS after administering an effective amount of a 19S complex botulinum toxin composition containing an animal protein.

In one embodiment, the time to reach the maximum DAS after administering the 19S complex botulinum toxin composition containing the animal protein to a mouse at a dose of 12 U/kg may be about 40% or more longer than the time to reach the maximum DAS after administering the present composition to a mouse at a dose of 12 U/kg. For example, the time to reach the maximum DAS after administering the 19S complex botulinum toxin composition containing the animal protein to a mouse at a dose of 12 U/kg may be about 20% or more, about 30% or more, about 40% or more, about 50% or more, about 30% to about 60%, about 30% to about 55%, about 35% to about 55%, or about 40% to 50% longer than the time to reach the maximum DAS after administering the composition to a mouse at a dose of 12 U/kg.

In the present specification, when referring to a value of data derived from a group consisting of two or more subjects, the corresponding value may mean an average value for the subjects. For example, when a data value derived from group A is "higher," "lower," "longer," "shorter," "larger," "smaller," "faster," or "slower" than a data value derived from group B, it may mean that an average value derived from subjects included in group A is "higher," "lower," "longer," "shorter," "larger," "smaller," "faster," or "slower" than an average value derived from subjects included in group B. However, it goes without saying that if meaning a value other than the average value is a conventional interpretation in the art, the conventional interpretation shall apply.

In the present specification, the DAS may be measured by administering an effective amount of the composition to a gastrocnemius muscle of a mouse and visually evaluating a degree of muscle paralysis of the mouse according to the criteria to be described later.

In one embodiment, the 19S complex botulinum toxin composition containing an animal protein may comprise albumin (for example, human serum albumin) as the animal protein. The 19S complex botulinum toxin composition containing an animal protein may comprise an animal protein (for example, albumin) and sodium chloride as excipients.

In one embodiment, a time to reach a maximum DAS after administering the composition to a mouse at a dose of 12 U/kg may be 20 hours to 30 hours. For example, the time to reach the maximum DAS after administering the composition to a mouse at a dose of 12 U/kg may be about 18 hours to about 36 hours, about 20 hours to about 35 hours, about 20 hours to about 30 hours, about 20 hours to about 28 hours, about 22 hours to about 28 hours, about 23 hours to about 27 hours, about 24 hours to about 26 hours, or about 25 hours.

In one embodiment, a time required to reach a level of 75% compared to a baseline (pre-administration) CMAP (compound muscle action potential) value after administering an effective amount of the present composition may be shorter than a time required to reach a level of 75% compared to a baseline (pre-administration) CMAP value after administering an effective amount of the 19S complex botulinum toxin composition containing an animal protein.

In one embodiment, the time required to reach the level of 75% compared to the baseline CMAP value after administering the present composition to a rat at a dose of 4 U/kg (CMAP_{T75%}) may be less than 8 hours. For example, the CMAP_{T75%} after administering the present composition to a rat at a dose of 4 U/kg may be less than about 9 hours, less than about 8 hours, less than about 7.5 hours, about 7 hours to about 9 hours, about 7 hours to about 8.5 hours, about 7.5 hours to about 9 hours, about 7.5 hours to about 8.5 hours, or about 7.5 hours to about 8 hours, for example, about 7.8 hours.

In the present specification, the CMAP may be measured at the administered muscle site using a nerve and electromyography system (for example, model name UltraPro S100) after administering an effective amount of the composition to a gastrocnemius muscle of a rat. For example, the measurement may be performed by placing an anode on the right lumbar muscle at the 4th to 5th lumbar vertebrae, placing a cathode on the right sciatic nerve based on the 5th to 6th lumbar vertebrae, maintaining a distance of about 1 cm between the anode and the cathode, placing a recording electrode on the gastrocnemius muscle, placing a reference electrode on the Achilles tendon site, placing a ground electrode on the rectus femoris muscle site, setting a level and duration of stimulation to 20 to 30 mA and 0.2 ms, and setting a filter range of an amplifier to 2 to 10 kHz at 60 Hz.

In one embodiment, an area under the curve (CMAP_{AUC}) of a CMAP graph for 72 hours after administering an effective amount of the composition may be smaller than an area under the curve of a CMAP graph for 72 hours after administering an effective amount of a 19S complex botulinum toxin composition containing an animal protein.

In one embodiment, the area under the curve (CMAP_{AUC}) of the CMAP graph for 72 hours after administering an effective amount of the composition may be about 5% to about 15%, about 5% to about 12%, or about 7% to about 12%, for example, about 10% smaller than the area under the curve of the CMAP graph for 72 hours after administering an effective amount of the 19S complex botulinum toxin composition containing an animal protein.

In one embodiment, the area under the curve of the CMAP graph for 72 hours after administering the composition to a rat at a dose of 4 U/kg may be 1300 mV·hours or less. For example, the area under the curve of the CMAP graph for 72 hours after administering the composition to a rat at a dose of 4 U/kg may be 1350mV·hours or less, 1300 mV·hours or less, 1250 mV·hours or less, 1150 mV·hours to 1350 mV·hours, 1200 mV·hours to 1300 mV·hours, 1250 mV·hours to 1300 mV·hours, or 1200 mV·hours to 1250 mV·hours.

Conventionally, in the case of the 19S complex toxin, it takes several days or more for a therapeutic effect to be maximally manifested, which has caused a decrease in patient treatment satisfaction. However, according to an embodiment of the present invention, a subject administered with a therapeutically effective amount of the present composition, which does not contain an animal protein and comprises a non-complex botulinum toxin, can achieve a faster improvement or treatment of a disease or a symptom than when the same amount of the 19S complex toxin composition is administered to the same site, thereby increasing patient satisfaction.

Meanwhile, in order to release a botulinum toxin composition, a potency assay must be performed. As the potency assay method, a mouse LD₅₀ measurement method via a mouse potency assay (MPA) by intraperitoneal (IP) injection is currently used as the only potency assay method. According to an embodiment, when the present composition is administered to mice, a faster onset of efficacy is achieved compared to a non-complex botulinum toxin composition, so that a time required for the potency assay can be shortened, thereby improving an efficiency of a botulinum toxin manufacturing process.

In one embodiment, the composition may comprise a pharmaceutically acceptable excipient or additive. The pharmaceutically acceptable excipient or additive may be a stabilizer, an ionic compound, a surfactant, a buffer, a lyoprotectant, or a combination thereof, for example, an amino acid (for example, methionine), a salt (for example, NaCl), a buffer solution, a non-ionic surfactant (for example, a polysorbate, for example, polysorbate 20), a sugar (for example, a disaccharide and/or a monosaccharide, for example, glucose, fructose, galactose, trehalose, sucrose, and/or maltose), a polyol (for example, a sugar alcohol such as mannitol, inositol, lactitol, isomalt, xylitol, erythritol, sorbitol, and/or glycerol), or a combination thereof.

In one embodiment, the non-ionic surfactant may include, for example, a polyoxyethylene compound, an ethoxylated alcohol, an ethoxylated ester, an ethoxylated amide, a polyoxypropylene compound, a propoxylated alcohol, an ethoxylated/propoxylated block polymer and a propoxylated ester, an alkanolamide, an amine oxide, a fatty acid ester of a polyhydric alcohol, an ethylene glycol ester, a diethylene glycol ester, a propylene glycol ester, a glyceryl ester, a polyglyceryl fatty acid ester, a sorbitan ester, a sucrose ester, and/or a glucose (dextrose) ester. For example, it may include a polyoxyethylene fatty alcohol ester, a sorbitan fatty acid ester [Span], a polyoxyethylene sorbitan fatty acid ester [for example, polyoxyethylene (20) sorbitan monooleate (Tween 80 or polysorbate 80), polyoxyethylene (20) sorbitan monostearate (Tween 60 or polysorbate 60), polyoxyethylene (20) sorbitan monolaurate (Tween 20 or polysorbate 20), and other Tweens], a sorbitan ester, or a glycerol ester.

The botulinum toxin composition of the present invention may be formulated into any form, such as a solid or liquid formulation, for example, a lyophilized powder, a vacuum-dried powder, a liquid formulation, or a pre-filled syringe formulation.

The composition of one embodiment may be a vacuum-dried composition, a lyophilized composition, or a liquid composition.

In one embodiment, the botulinum toxin composition comprises a non-complex botulinum toxin, a surfactant, and/or an amino acid; and may comprise one or more components selected from the group consisting of a sugar, a sugar alcohol, and an ionic compound.

The surfactant may be a non-ionic surfactant, for example, an alkylene oxide-based surfactant such as an ethylene oxide-based surfactant or a propylene oxide-based surfactant. For example, it may be a polyoxyethylene sorbitan fatty acid ester and/or a polyoxyethylene-polyoxypropylene block copolymer, for example, a poloxamer and/or a polysorbate.

In one embodiment, the polysorbate may include, for example, polysorbate 20, 40, 60, 80, or 100, and for example, may include polysorbate 20. In one embodiment, the amino acid may be at least one selected from the group consisting of methionine, arginine, alanine, glutamic acid, aspartic acid, glycine, and cysteine.

In one embodiment, the amino acid may comprise a natural amino acid (one or more of the 20 known natural amino acids, or one or more of the 22 natural amino acids including selenocysteine and pyrrolysine). For example, the amino acid may comprise at least one selected from the group consisting of methionine, isoleucine, alanine, tryptophan, tyrosine, glutamic acid, aspartic acid, arginine, glycine, and cysteine, and for example, may comprise methionine.

For example, the composition of one embodiment may comprise a polysorbate (for example, polysorbate 20) and methionine as a stabilizer.

In one embodiment, the sugar may comprise trehalose, sucrose, maltose, fructose, raffinose, lactose, or glucose. The sugar alcohol may comprise cyclodextrin, mannitol, sorbitol, glycerol, xylitol, or inositol. For example, the lyophilized composition of one embodiment may comprise sucrose.

In one embodiment, the ionic compound may comprise sodium chloride, sodium phosphate, ammonium phosphate, magnesium sulfate, sodium acetate, sodium lactate, sodium succinate, sodium propionate, or potassium phosphate.

In one embodiment, the lyophilized composition comprises a non-complex botulinum toxin, a surfactant, and/or an amino acid; and may comprise one or more components selected from the group consisting of a sugar, a sugar alcohol, and an ionic compound. For example, it may comprise a non-complex botulinum toxin, a non-ionic surfactant, an amino acid, a sugar or a sugar alcohol, and an ionic compound. For example, it may comprise a non-complex botulinum toxin; a poloxamer or a polysorbate; at least one selected from the group consisting of methionine, arginine, alanine, glutamic acid, aspartic acid, glycine, and cysteine; a sugar or a sugar alcohol; and an ionic compound.

In one embodiment, the liquid composition may comprise a non-complex botulinum toxin, a surfactant, an amino acid, and/or an ionic compound. For example, it may comprise a non-complex botulinum toxin; a poloxamer or a polysorbate; at least one selected from the group consisting of methionine, arginine, alanine, glutamic acid, aspartic acid, glycine, and cysteine; and an ionic compound. A pH of the liquid composition of one embodiment may be 5.5 to 7.0.

In one embodiment, the composition may be for improving skin wrinkles, square jaw, pointed chin, wounds, skin softening, scars, acne, pores, elasticity, or keloid symptoms, or for treating at least one disease selected from the group consisting of facial spasm, blepharospasm, torticollis, cervical dystonia, oropharyngeal dystonia, spasmodic dysphonia, migraine, pruritus ani, and hyperhidrosis.

According to an embodiment of the present invention, the above-described botulinum toxin may comprise botulinum toxin type A. For example, it may comprise a botulinum toxin type A subtype, such as an A1, A2, A3, A4, A5, A6, A7, A9, or A10 toxin. The botulinum toxin type A may comprise a chimeric toxin. For example, a light chain and/or a heavy chain of the botulinum toxin type A may comprise a combination of the first to fourth domains of, for example, the A1, A2, A3, A4, A5, A6, A7, A9, and/or A10 toxins. For example, any one of the light chain and/or the heavy chain of the botulinum toxin type A may comprise a combination of the first to fourth domains of the A1, A2, A3, A4, A5, A6, A7, A9, and/or A10 toxins, and the other one may comprise a light chain or a heavy chain of a domain of types B to H toxins.

According to an embodiment of the present invention, there is provided a method for shortening a time required for measuring a potency of a botulinum toxin, the method comprising administering the above-described botulinum toxin composition of the present disclosure to a mouse to measure an LD₅₀ of the composition of the present disclosure, wherein the time required for measuring the LD₅₀ is shorter than a time required for measuring an LD₅₀ by administering a 19S complex botulinum toxin composition containing an animal protein to a mouse.

According to an embodiment of the present invention, in a method for shortening a time required for measuring a potency of a botulinum toxin in a mouse, there is provided a use of the above-described botulinum toxin composition.

According to an embodiment of the present invention, there is provided a method for treating a disease of a subject, the method comprising locally administering a therapeutically effective amount of the above-described botulinum toxin composition to the subject in need thereof.

According to an embodiment of the present invention, there is provided a use of an effective amount of the botulinum toxin formulation in the manufacture of a medicament for treating a disease.

According to an embodiment of the present invention, there is provided a method for rapidly improving or treating a disease or a symptom of a subject, the method comprising locally administering a therapeutically effective amount of the above-described botulinum toxin composition to the subject in need thereof.

Hereinafter, the present invention will be described in more detail with reference to examples, but these are only for illustrating the present invention and are not intended to limit the scope thereof in any way.

### Example 1: Non-Clinical Efficacy Test 1 of Non-Complex Botulinum Toxin and Complex Botulinum Toxin

Animals: Female 5-week-old CD1 (ICR) mice were purchased from Orient Bio Inc., and after 1 week of acclimation and quarantine, the 6-week-old mice were used in the experiment. Sterilized solid feed for laboratory animals (R40-10, SAFE, France) and drinking tap water sterilized at high temperature and high pressure were provided *ad libitum*. During the acclimation, quarantine, and experimental periods, the mice were housed under specific-pathogen-free conditions set at a temperature of 23 ± 3°C, a relative humidity of 55 ± 15%, a 12-hour light cycle (8:00 AM to 8:00 PM), a ventilation rate of 15 times/hour, and an illuminance of 150 to 300 Lux. This study was conducted after review and approval (A-2022-007) by the Institutional Animal Care and Use Committee (IACUC) of Medytox Inc.

Test Substances: Coretox (Lot No.: E623001, Medytox), MBA-P01 (Lot No.: TFXA21004, Medytox), and NXA20001 (Medytox) listed in Table 1 were used.

**[Table 1]**

| Test Group | Product Name or Code Name | Manufacturer | Botulinum Toxin | Excipient |
|---|---|---|---|---|
| Albumin-free BoNT/A (7S) | Coretox | Medytox | Approx. 150 kDa, non-complex botulinum toxin type A | Methionine, Polysorbate 20, Sucrose, Sodium chloride |
| Albumin BoNT/A (19S) | MBA-P01 | Medytox | Approx. 900 kDa, complex botulinum toxin type A | Human serum albumin, Sodium chloride |
| Albumin-free BoNT/A (19S) | NXA20001 | Medytox | Approx. 900 kDa, complex botulinum toxin type A | Methionine, Polysorbate 20, Sucrose, Sodium chloride |

Each test substance was diluted to a concentration of 60 U/mL using sterile normal saline (Dai Han Pharm. Co., Ltd.).

The day on which the test substance was administered was designated as Day 0. After anesthetizing the mice using an injectable anesthetic (100 mg/kg ketamine hydrochloride + 10 mg/kg xylazine), each test substance was administered to the right gastrocnemius muscle of the mice at a dose of 0.2 mL/kg using a Hamilton syringe according to the group configuration shown in Table 2.

**[Table 2]**

| Group | Number of Animals | Test Group | Dose (U/kg) | Route of Administration | Evaluation Index |
|---|---|---|---|---|---|
| 1 | 6 | Albumin-free BoNT/A (7S) | 12 | Right gastrocnemius muscle | DAS |
| 2 | 16 | Albumin BoNT/A (19S) | 12 | Right gastrocnemius muscle | DAS |
| 3 | 10 | Albumin-free BoNT/A (19S) | 12 | Right gastrocnemius muscle | DAS |

For evaluation, the digit abduction score (DAS) assay, which visually evaluates the degree of muscle paralysis in mice, was used. The DAS values shown in Table 3 represent the degree of muscle paralysis determined by the shape of the toes on the administered side of the mice.

**[Table 3]**

| Score | Criteria |
|---|---|
| 0 | Normal; no difference from the shape of the foot on the non-injected side |
| 1 | The width between the toes is narrowed, or two toes are stuck together and the rest are fully extended |
| 2 | The width between all toes is significantly narrowed, or three toes are stuck together |
| 3 | The foot is curved and four toes are stuck together |
| 4 | The foot is curved and all toes are stuck together |

Statistics: GraphPad Prism 7.05 (GraphPad Software Inc., CA, USA) was used for graph presentation, and SPSS software 25.0 (SPSS Inc., IL, USA) and Excel (2013, MS, USA) were used for statistical analysis. The experimental results were expressed as mean ± standard deviation. For statistical analysis between the two groups, a two-tailed t-test was performed, and a p-value of less than 0.05 was considered statistically significant.

### Results

Referring to FIG. 1, three different botulinum toxin formulations were administered to the right gastrocnemius muscle of mice at a dose of 12 U/kg, and then DAS was evaluated over time. As a result of the evaluation, until about 25 hours post-administration, which is the time period when the BoNT/A (7S) albumin-free formulation reaches the maximum DAS score, a trend of an increase in the DAS score was observed in the group administered with the BoNT/A (7S) albumin-free formulation compared to the groups administered with the BoNT/A (19S) albumin formulation and albumin-free formulation. In particular, at 8 hours post-administration, a significantly increased DAS score was observed in the group administered with the BoNT/A (7S) albumin-free formulation compared to the groups administered with the BoNT/A (19S) albumin formulation and albumin-free formulation, confirming that the onset of efficacy of the BoNT/A (7S) albumin-free formulation is faster.

In order to more clearly confirm the fast-onset effect identified above, the time to reach the maximum DAS (T_{DASmax}) was analyzed and is shown in FIG. 2. As a result, the average was 25 hours in the group administered with the BoNT/A (7S) albumin-free formulation, whereas a significant increase of about 50% or more was observed in the case of the BoNT/A (19S) albumin formulation (average 37.9 hours) and the BoNT/A (19S) albumin-free formulation (38.4 hours) compared to the group administered with the BoNT/A (7S) albumin-free formulation. From these results, the fast-onset effect was confirmed in the non-animal and non-complex botulinum toxin type A formulation compared to the animal and non-animal complex botulinum toxin type A formulations.

### Example 2: Non-Clinical Efficacy Test 2 of Non-Complex Botulinum Toxin and Complex Botulinum Toxin

Through the following experiment, it was confirmed that the fast-onset effect of the non-complex and albumin-free botulinum toxin type A formulation also appeared identically in a rat model. Female 6-week-old SD rats were purchased from Orient Bio Inc., and after 1 week of acclimation and quarantine, the 7-week-old rats were used in the experiment. Sterilized solid feed for laboratory animals (R40-10, SAFE, France) and drinking tap water sterilized at high temperature and high pressure were provided ad libitum. During the acclimation, quarantine, and experimental periods, the rats were housed under specific-pathogen-free conditions set at a temperature of 23 ± 3°C, a relative humidity of 55 ± 15%, a 12-hour light cycle (8:00 AM to 8:00 PM), a ventilation rate of 15 times/hour, and an illuminance of 150 to 300 Lux. This study was conducted after review and approval (A-2023-001) by the Institutional Animal Care and Use Committee (IACUC) of Medytox Inc.

The test substances were prepared as follows. Coretox (Medytox), a finished product of a 100-unit non-complex and albumin-free botulinum toxin type A, and MBA-P01 (Medytox), a finished product of a complex and albumin botulinum toxin type A, were used [Table 1]. Each test substance was diluted to a concentration of 40 U/mL using sterile normal saline (Dai Han Pharm. Co., Ltd.).

The day on which the test substance was administered was designated as Day 0. After anesthetizing the rats using an injectable anesthetic (60 mg/kg ketamine hydrochloride + 10 mg/kg xylazine), each test substance was administered to the right gastrocnemius muscle of the rats at a dose of 0.1 mL/kg using a Hamilton syringe according to Table 4 below.

**[Table 4]**

| Group | Number of Animals | Test Group | Dose (U/kg) | Route of Administration | Evaluation Index |
|---|---|---|---|---|---|
| 1 | 8 | Albumin-free BoNT/A (7S) | 4 | Right gastrocnemius muscle | DAS, CMAP |
| 2 | 8 | Albumin BoNT/A (19S) | 4 | Right gastrocnemius muscle | DAS, CMAP |

As in Example 1, the digit abduction score (DAS) assay (Table 3), which visually evaluates the degree of rat muscle paralysis, was used. In addition, evaluation was performed using a compound muscle action potential (CMAP) assay. CMAP was measured over time post-administration of the test substance in the right gastrocnemius muscle (administered muscle) region of each rat using an UltraPro S100 (Natus Neurology Inc.) device. After anesthesia with an injectable anesthetic, the hair at the measurement site was shaved, and the rats were placed in a prone position. The anode was placed on the right lumbar muscle of the 4th to 5th lumbar vertebrae of the rat, and the cathode was placed on the right sciatic nerve based on the 5th to 6th lumbar vertebrae. The distance between the anode and the cathode was maintained at about 1cm. The recording electrode was placed on the gastrocnemius muscle, the reference electrode was placed on the Achilles tendon region, and the ground electrode was placed on the rectus femoris muscle region. The stimulation level and duration were set to 20 to 30 mA and 0.2 ms, respectively, and the filter range of the amplifier was set to 2-10 K at 60 Hz. When measured under these conditions, the height from the baseline to the peak after the latency of the waveform was digitized as the CMAP measurement value. The obtained data were statistically processed in the same manner as in Example 1, and the area under the curve (AUC) of the CMAP graph was calculated using GraphPad Prism 7.05 (GraphPad Software Inc., CA, USA).

### Results

Referring to FIG. 3, two different formulations of botulinum toxin type A were administered to the right gastrocnemius muscle of rats at a dose of 4 U/kg, and then DAS was evaluated over time. As a result of the evaluation, at 24 hours post-administration, the expression of a DAS score of 1 was observed in all 8 individuals (100%) in the group administered with the BoNT/A (7S) albumin-free formulation, whereas a lower expression was observed in the group administered with the BoNT/A (19S) albumin formulation, where a score of 1 was observed in only 5 out of 8 rats (62.5%). Furthermore, a trend of an increase in the average score at 24 hours was also observed in the group administered with the BoNT/A (7S) albumin-free formulation.

Referring to FIG. 4, as a result of measuring CMAP over time post-administration, a trend of a decrease in the CMAP score was observed in the group administered with the BoNT/A (7S) albumin-free formulation compared to the group administered with the BoNT/A (19S) albumin formulation. In order to more clearly confirm the fast-onset effect identified above, the time to reach a level of 75% relative to the pre-administration CMAP value (CMAP_{T75%}) and the AUC of the CMAP graph for 72 hours (CMAP_{AUC}) were calculated and analyzed. Referring to FIG. 5, it was confirmed that the time to reach a level of 75% relative to the pre-administration CMAP value was significantly lower in the group administered with the BoNT/A (7S) albumin-free formulation compared to the group administered with the BoNT/A (19S) albumin formulation. In addition, a significantly decreased CMAP_{AUC} value was observed in the group administered with the BoNT/A (7S) albumin-free formulation compared to the group administered with the BoNT/A (19S) albumin formulation. From these results, the fast-onset effect of the non-animal and non-complex botulinum toxin type A formulation was also confirmed in the rat model.

## Claims

1. A botulinum toxin composition for use in a method for treating a disease in a subject in need thereof, the composition being free of animal protein and comprising a non-complexed botulinum toxin as an active ingredient, wherein the method comprises locally administering a therapeutically effective amount of the composition to the subject in need thereof, and wherein the composition has a faster onset of efficacy than a composition containing animal protein and comprising a 19S botulinum toxin complex as an active ingredient.

2. The composition of claim 1, wherein a time to reach maximum DAS after administration of an effective amount of the composition is shorter than a time to reach maximum DAS after administration of an effective amount of the 19S botulinum toxin complex composition containing animal protein.

3. The composition of claim 2, wherein a time to reach maximum DAS after administering the 19S botulinum toxin complex composition containing animal protein to a mouse at a dose of 12 U/kg is at least 40% longer than a time to reach maximum DAS after administering the composition to a mouse at a dose of 12 U/kg.

4. The composition of claim 2, wherein the time to reach maximum DAS after administering the composition to a mouse at a dose of 12 U/kg is 20 hours to 30 hours.

5. The composition of claim 1, wherein a time required to reach a level of 75% relative to a pre-administration CMAP value after administering an effective amount of the composition is shorter than a time required to reach a level of 75% relative to a pre-administration CMAP value after administering an effective amount of the 19S botulinum toxin complex composition containing animal protein.

6. The composition of claim 5, wherein the time required to reach a level of 75% relative to the pre-administration CMAP value after administering the composition to a rat at a dose of 4 U/kg is less than 8 hours.

7. The composition of claim 1, wherein an area under the curve of a CMAP graph for 72 hours after administration of an effective amount of the composition is smaller than an area under the curve of a CMAP graph for 72 hours after administration of an effective amount of the 19S botulinum toxin complex composition containing animal protein.

8. The composition of claim 7, wherein the area under the curve of the CMAP graph for 72 hours after administering the composition to a rat at a dose of 4 U/kg is 1300 mV*hours or less.

9. The composition of any one of claims 1 to 8, wherein the composition comprising the botulinum toxin, and further comprising one or more components selected from the group consisting of a surfactant, an amino acid, and an ionic compound.

10. The composition of claim 9, wherein the surfactant is at least one selected from the group consisting of polysorbate or poloxamer, and the amino acid is at least one selected from the group consisting of methionine, arginine, alanine, glutamic acid, aspartic acid, arginine, glycine, or cysteine.

11. The composition of any one of claims 1 to 8, wherein the composition is for improving skin wrinkles, square jaw, pointed jaw, wounds, skin softening, scars, acne, pores, elasticity, or keloid symptoms, or for treating at least one disease selected from the group consisting of facial spasm, eyelid spasm, torticollis, blepharospasm, cervical dystonia, mid-pharyngeal dystonia, spasmodic dysphonia, migraine, pruritus ani, or hyperhidrosis.

12. The composition of any one of claims 1 to 8, wherein the botulinum toxin is serotype A.
